# EUROPEAN PATENT SPECIFICATION

(11) **EP 3 646 806 B1**
(45) Date of publication and mention of the grant of the patent: **22.03.2023**
(21) Application number: 18824650.8
(22) Date of filing: 08.06.2018
(51) Int. Cl.: A61B 17/22, A61B 17/3207

(54) **MECHANICAL THROMBUS REMOVAL DEVICE**
VORRICHTUNG ZUR MECHANISCHEN THROMBUSENTFERNUNG
DISPOSITIF MÉCANIQUE D'ÉLIMINATION DE THROMBUS

(30) Priority: 30.06.2017 CN 201710525498
(43) Date of publication of application: 06.05.2020
(73) Proprietor: Shanghai Bluevascular Medtech Co., Ltd., Shanghai 201321 (CN)
(72) Inventor: HAN, Jianchao, Shanghai 201318 (CN); ZHANG, Linlin, Shanghai 201318 (CN); DING, Shuangxi, Shanghai 201318 (CN); SHI, Zengzuo, Shanghai 201318 (CN); FAN, Yaming, Shanghai 201318 (CN); LI, Zhonghua, Shanghai 201318 (CN); MIAO, Zhenghua, Shanghai 201318 (CN)
(74) Representative: Lavoix
(86) International application number: PCT/CN2018/090482
(87) International publication number: WO 2019/001245

(56) References cited:
- EP-A1- 2 698 119
- EP-A2- 0 353 087
- WO-A1-00/76410
- CN-A- 104 323 823
- CN-A- 104 586 469
- CN-A- 104 586 469
- CN-U- 201 743 732
- US-A- 5 306 244
- US-A- 5 496 267
- US-A1- 2005 240 146
- US-A1- 2007 250 096
- US-A1- 2011 160 758
- US-A1- 2015 173 782
- US-B1- 9 211 163

## Description

### TECHNICAL FIELD

The present invention relates to the technical field of medical instruments and, more specifically, to a mechanical thrombectomy apparatus.

### BACKGROUND

Deep venous thrombosis (DVT) in lower extremities is a disease caused by abnormal clotting of blood in deep veins of lower extremities. DVT leads to an increased venous pressure, blocked blood flow, swelling, pain and dysfunction of the lower extremities, and a risk of a detached thrombus. The detached thrombus may be brought to a pulmonary artery by the bloodstream to cause pulmonary embolism (PE). If no effective treatment is received in the acute phase of DVT, post thrombosis syndrome (PTS) including thrombus organization, venous obstruction, valvular dysfunction, venous reflux, and venous hypertension may occur, which endangers the survival of the extremities and is life-threatening.

Aspirin, heparin, warfarin or the like, taken orally or injected, can enter the blood system to effectively dissolve a venous thrombus, thereby significantly reducing the incidence of DVT and PE. Meanwhile, medical prevention and treatment also have significant limitations. As for hemorrhagic constitutions, such drugs may cause severe bleeding and endanger the life of a patient in circumstances such as hemorrhagic diabetes, hemorrhagic apoplexy, neurosurgical operation, severe trauma, hemothorax, pelvic and lower-extremity fracture caused from intracranial hemorrhage, and anticoagulation disorder.

Catheter-directed thrombolysis (CDT) enables targeted delivery of a thrombolytic drug (such as urokinase) to the site of a thrombus, thereby effectively reducing the load of the thrombus, realizing early recovery of forward blood flow in a vein, eliminating or relieving venous obstruction, protecting the morphology and function of venous valves, blocking the pathological process of PTS, and reducing the incidence of PTS. However, the CDT treatment on DVT takes a long time for thrombolysis (53.4 hours on average), which leads to long-term indwelling of a catheter, increased patient discomfort, and prolonged hospitalization. CDT requires repeated blood draw, close monitoring, and high-level care. It is unable to restore blood flow as quickly as possible in the cases that the swelling is severe and the survival of the extremities is endangered. The thrombolytic drug used in CDT may induce bleeding. CDT is not suitable for patients with a high risk of bleeding (such as patients with severe hypertension), nor for postpartum and pregnant patients.

Conventional surgical thrombectomy is suitable for patients who suffer clinically severe DVT and cannot take thrombolytic drugs. It has the following disadvantages: the surgical venous thrombectomy is an invasive manipulation and is not suitable for patients with poor general conditions; the thrombectomy may cause valvular dysfunction; and further thrombolysis and anticoagulation against residual thrombi may easily cause wound complications.

With the development of technologies, a percutaneous mechanical thrombectomy (PMT) apparatus has been developed in recent years. It is a set of instruments for treating acute and subacute thrombosis in blood vessels. The apparatus removes thrombi in blood vessels by means of dissolution, fragmentation, and aspiration, to restore blood circulation and valvular function. PMT is an interventional intracavitary thrombectomy apparatus capable of quickly removing blood clots and restoring blood flow and valvular function.

US patent publication US2014/0088610 A1 discloses a structure of a mechanical thrombectomy apparatus, which has a catheter with multiple side holes on its tip. An independent conduit for liquid delivery and perfusion is provided in the catheter, and the conduit is formed with multiple orifices on its tip. When the apparatus starts working, high-pressure saline is perfused into the independent conduit and ejected from the orifices on the tip of the conduit. Bernoulli Effect is produced by interaction between the high-speed saline jets and the side holes on the tip of the catheter, and thus thrombi are aspirated into the catheter via the side holes. Meanwhile, large pieces of the aspirated thrombi are fragmented by the high-speed saline jets, and are expelled through aspiration by a negative pressure. After the thrombectomy procedure, thrombolytic drugs can be perfused through the catheter into the blood vessels to reduce residual thrombi. This patent solves the problem of thrombus aspiration and expelling. However, due to its design features, the high-speed saline jets may be ejected via the side holes on the catheter and destroy red blood cells in the blood. Since not all of these destroyed red blood cells are expelled through aspiration, complications such as hematuria and renal failure may easily occur. The high-speed saline jets stimulate the vascular wall and may easily cause nervous breakdown. Since perfusion of the thrombolytic drugs after the thrombectomy procedure is carried out via a passage same as the passage used in thrombus aspiration, a part of the thrombi aspirated into the catheter may again enter the body of a patient along with drug perfusion, which reduces the thrombectomy effect.

Patent publication WO2011/024124 A1 discloses a structure of a mechanical thrombectomy apparatus, which has a catheter with side holes on its tip and a spiral structure inside the catheter. When the apparatus starts working, thrombi near the side holes on the catheter are aspirated into the catheter under a negative pressure formed by high-speed rotation of the spiral structure. The spiral structure cooperates with the side holes on the catheter to cut the aspirated thrombi, thereby achieving the purpose of thrombus fragmentation. Finally, the thrombi are expelled. This apparatus carries out thrombus fragmentation inside the catheter, thus avoiding hematuria. However, since the spiral structure rotates at an extremely high speed during aspiration, the temperature in the catheter may easily grow too high, which will lead to breakage of a guidewire and cause adverse reactions. Due to its design features, perfusion of thrombolytic drugs cannot be carried out in the apparatus.

Document US 2005/240146 discloses a device suitable for removing material from a living being, featuring an infusate pump, and an aspiration pump, both powered by a motor, wherein the aspiration pump and infusate pump preferably feature a helical pumping mechanism, and operate at a high rate of rotation, thereby ensuring adequate pumping performance and flexibility.

Document US 2015/173782 discloses a system of devices for treating an artery which includes an arterial access sheath adapted to introduce an interventional catheter into an artery and an elongated dilator positionable within the internal lumen of the sheath body.

Document US 9 211 163 discloses a method and apparatus for the evacuation of intracerebral hematomas comprising a minimally invasive non-operating room surgical apparatus within a neuro-navigation system that can provide real-time imaging of the ICH evacuation procedure.

Document CN 104 586 469 discloses a local circulation type thrombus removing device mainly comprising an impeller assembly used for smashing the thrombus, a head end protective pipe, an inner pipe, an outer pipe, a first Y-shaped valve and a second Y-shaped valve.

Persons skilled in the art have been seeking for solutions to eliminate the defects of the existing mechanical thrombectomy apparatuses.

### SUMMARY OF THE DISCLOSURE

An objective of the present invention is to provide a mechanical thrombectomy apparatus, to eliminate the defects of the mechanical thrombectomy apparatuses in the prior art.

To solve the foregoing technical problem, the present invention provides a mechanical thrombectomy apparatus according to the appended set of claims.

In the mechanical thrombectomy apparatus provided by the present invention, the thrombus aspiration system includes the aspiration device and the thrombus aspiration tube. The thrombus aspiration system of the present disclosure provides a negative pressure in a manner different from an existing manner of generating a negative pressure via siphoning by high-speed rotation of a spiral structure. Thus, the negative pressure required for thrombus aspiration can be generated without high-speed rotation. In this way, the problem of breakage of a guidewire caused by over-high temperature resulting from extremely high-speed rotation of the spiral structure in the existing process of generating a negative pressure can be effectively solved. In addition, the thrombus aspiration system and the thrombus fragmentation system of the present disclosure are two mutually independent systems, which effectively prevents thrombus aspiration and thrombus fragmentation processes from influencing one another. The thrombus fragmentation process is carried out in the thrombus removal head, which effectively reduces the probability of incurring hematuria and renal failure complications and reduces the incidence of vascular perforation. The present disclosure also adopts the independent drug perfusion system.

Hence, the thrombolytic drug can be perfused to the site of a lesion at any time during the operation. Therefore, the problem caused by the circumstance that the thrombus already aspirated into the tube again enters the body of the patient along with drug perfusion can be avoided. The present disclosure also makes improvement on the thrombus removal head to increase the thrombolysis area and improve the thrombolysis efficiency. The mechanical thrombectomy apparatus provided by the present disclosure integrates perfusion of a thrombolytic drug, thrombus aspiration by a negative pressure, and mechanical thrombus fragmentation, which can significantly accelerate the thrombectomy procedure, reduce the operation time, relieve pain from the patient, and expand the application range of the surgery.

### BRIEF DESCRIPTION OF THE DRAWINGS

FIG. 1 is a schematic structural diagram of a mechanical thrombectomy apparatus according to an embodiment of the present invention;
FIG. 2 is a schematic structural diagram of a thrombus removal head according to an embodiment of the present invention;
FIG. 3 is a schematic cross-sectional diagram along A-A direction in FIG. 2;
FIG. 4 is a schematic structural diagram of a thrombus fragmentation cutter according to an embodiment of the present invention;
FIG. 5 is a schematic cross-sectional diagram of a sheath connector according to an embodiment of the present invention;
FIG. 6 is a schematic cross-sectional diagram of a rear-end connector of a transmission tube according to an embodiment of the present invention;
FIG. 7 is a schematic partial cross-sectional diagram of the transmission tube according to an embodiment of the present invention;
FIG. 8 is a schematic diagram illustrating a configuration of a rotation actuator according to an embodiment of the present invention; and
FIG. 9 is a schematic diagram illustrating another configuration of the rotation actuator according to an embodiment of the present invention.

In the drawings:
1000 denotes a mechanical thrombectomy apparatus; 1340 denotes a peristaltic pump; 1310 denotes a flexible tube; 1320 denotes a pump head; 1120 denotes a sheath; 1110 denotes a thrombus removal head; 1111 denotes a top hole; 1112 denotes an inclined hole; 1113 denotes a thrombus removal side hole; 1330 denotes a collection vessel; 1270 denotes a transmission tube; 1221 denotes a first side hole; 1251 denotes a perfusion hole; 1220 denotes a front-end transmission tube; 1240 denotes an intermediate transmission tube; 1250 denotes a rear-end transmission tube; 1260 denotes an inner tube; 1140 denotes a rotation actuator; 1141 denotes a rotary motor; 1252 denotes a first gear; 1142 denotes a second gear; 1150 denotes a rear-end connector of the transmission tube; 1210 denotes a thrombus fragmentation cutter; 1211 denotes a blade; 1180 denotes an injector; 1160 denotes an infusion tube; 1170 denotes a check valve; 1130 denotes a sheath connector; 1230 denotes a positioning ring; 1131, 1151 denote a fixing cap; 1132, 1152 denote a seal ring; 1153 denotes a locking cap; 1154 denotes a seal collar; 1155 denotes a guidewire hole.

### DETAILED DESCRIPTION

The mechanical thrombectomy apparatus provided by the present invention is further described in detail below with reference to specific embodiments and the accompanying drawings. The advantages and features of the present invention become clearer according to the following description and the appended claims. It should be noted that, the accompanying drawings are simplified and not depicted in exact proportion, and are merely provided for conveniently and explicitly illustrating the objective of the embodiments of the present invention.

The definitions of "proximal end" and "distal end" in the text are: "distal end" generally referring to an end of a medical instrument that first enters the body of a patient during a normal operation, and "proximal end" generally referring to an end of a medical instrument that is close to an operator during a normal operation.

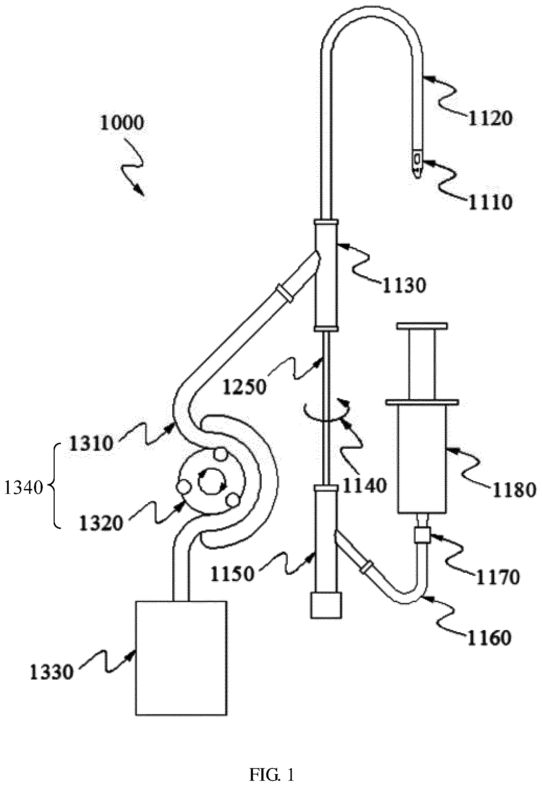

FIG. 1 is a schematic structural diagram of a mechanical thrombectomy apparatus according to the present invention. The mechanical thrombectomy apparatus 1000 includes: a thrombus removal head 1110, a thrombus aspiration system, and a thrombus fragmentation system. The thrombus removal head 1110 is connected to a sheath connector 1130 through a sheath 1120. The thrombus aspiration system includes an aspiration device and a thrombus aspiration tube. A proximal end of the thrombus aspiration tube is connected to the aspiration device, and a distal end of the thrombus aspiration tube is connected to the sheath connector 1130. A cavity, defined by a transmission tube of the thrombus fragmentation system and the sheath 1120, is communicated with the thrombus aspiration tube to form a thrombus aspiration lumen.

In the thrombus aspiration system of the invention, the aspiration device is a peristaltic pump 1340, and the thrombus aspiration tube is a flexible tube 1310. The thrombus aspiration system further includes a collection vessel 1330 for collecting thrombi. A distal end of the flexible tube 1310 is communicated with a proximal end of the sheath 1120, a proximal end of the flexible tube 1310 is communicated with the collection vessel 1330, and a distal end of the sheath 1120 is communicated with the thrombus removal head 1110. Under a negative pressure produced by the peristaltic pump 1340, thrombi aspirated by the thrombus removal head 1110 are delivered into the collection vessel 1330 through the sheath 1120 and the flexible tube 1310. In addition, the peristaltic pump 1340 further includes a pump head 1320. The flexible tube 1310 is mounted on the pump head 1320 and is switched between a squeezed state and a non-squeezed state along with rotation of the pump head 1320.

The peristaltic pump 1340 works in a way similar to squeezing a fluid-filled flexible tube 1310 with fingers, and the fluid in the tube moves forward as the fingers slide forward. The peristaltic pump 1340 follows the same principle, while the fingers are replaced by the pump head 1320. The pump head 1320 alternately squeezes and releases the flexible tube 1310 to pump the fluid. When the pump head 1320 squeezes the flexible tube 1310, a negative pressure is produced by the tension of the flexible tube 1310 with the movement of the pump head 1320, and the fluid is forced to flow by the negative pressure. In the present example, the fluid in the flexible tube 1310 consists of thrombi and/or blood. Since the negative pressure is produced by the peristaltic pump, the rotation speed of the pump head 1320 is not required to be high. Therefore, compared with an existing manner of generating a negative pressure, required for thrombus aspiration, via siphoning by high-speed rotation of a spiral structure, the temperature rise during the process of generating the negative pressure is largely reduced, and the problem of breakage of a guidewire caused by over-high temperature is avoided. The flexible tube 1310 is preferably a silicone catheter, and the collection vessel 1330 is preferably a collection bag. In the present example, the manner of providing a negative pressure includes, but not limited to, using a peristaltic pump, and persons skilled in the art can provide a negative pressure for removing thrombi by using a negative pressure drainage bag or the like.

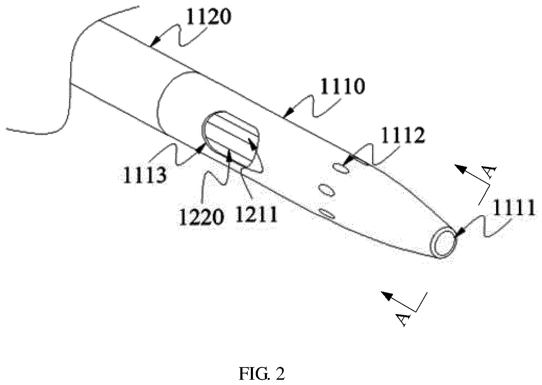

The thrombus fragmentation system includes: a rotation actuator 1140, a transmission tube 1270, and a thrombus fragmentation cutter 1210. The transmission tube 1270 and the thrombus fragmentation cutter 1210 are not shown in FIG. 1, and will be described in detail below with reference to FIGs. 2-7. Specifically, a proximal end of the transmission tube 1270 is connected to the rotation actuator 1140, a distal end of the transmission tube 1270 is connected to the thrombus fragmentation cutter 1210, and the transmission tube 1270 extends into the thrombus removal head 1110 along the lumens of the sheath connector 1130 and the sheath 1120. The rotation actuator 1140 enables the transmission tube 1270 to rotate and drive the thrombus fragmentation cutter 1210 to rotate, so as to fragment a thrombus aspirated into the thrombus removal head 1110.

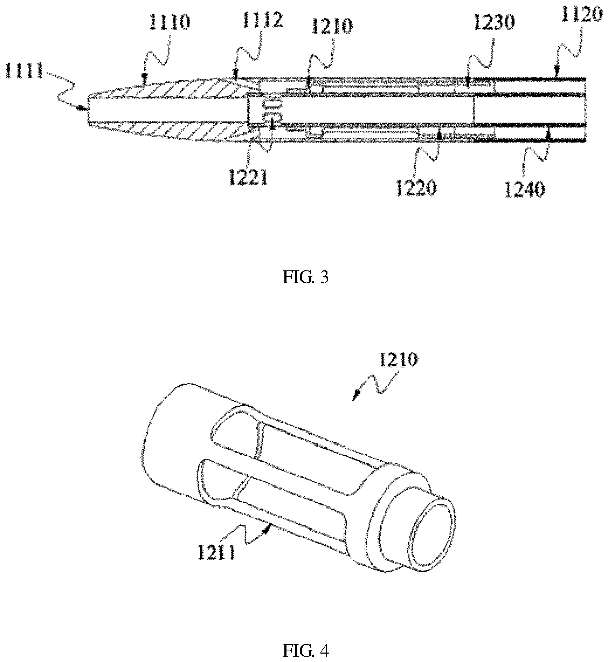

Referring to FIG. 3 and FIG. 4 in the present embodiment, FIG. 4 is a schematic structural diagram of the thrombus fragmentation cutter. As shown in FIG. 4, the thrombus fragmentation cutter 1210 is in a hollow-out tubular structure and includes at least one blade 1211. The thrombus fragmentation cutter 1210 is placed in the lumen of the thrombus removal head 1110, and the location of the blade 1211 is corresponding to the location of a thrombus removal side hole 1113 on the thrombus removal head 1110.

In the present invention, the mechanical thrombectomy apparatus 1000 further includes a drug perfusion system. The drug perfusion system includes an injector 1180 and an infusion tube 1160. A proximal end of the infusion tube 1160 is connected to the injector 1180, a distal end of the infusion tube 1160 is communicated with the transmission tube 1270 through the sheath connector 1130, or the distal end of the infusion tube is communicated with the transmission tube through a rear-end connector of the transmission tube. A thrombolytic drug loaded in the injector 1180 passes through the transmission tube 1270 and the thrombus removal head 1110 to reach the site of a thrombus, and then softens or dissolves the thrombus on the site. Preferably, the drug perfusion system further includes a check valve 1170 which is mounted on the infusion tube 1160 to control the flow direction of the liquid delivered in the infusion tube 1160. The distal end of the infusion tube is communicated with the transmission tube through the sheath connector, or the distal end of the infusion tube is communicated with the transmission tube through the rear-end connector of the transmission tube.

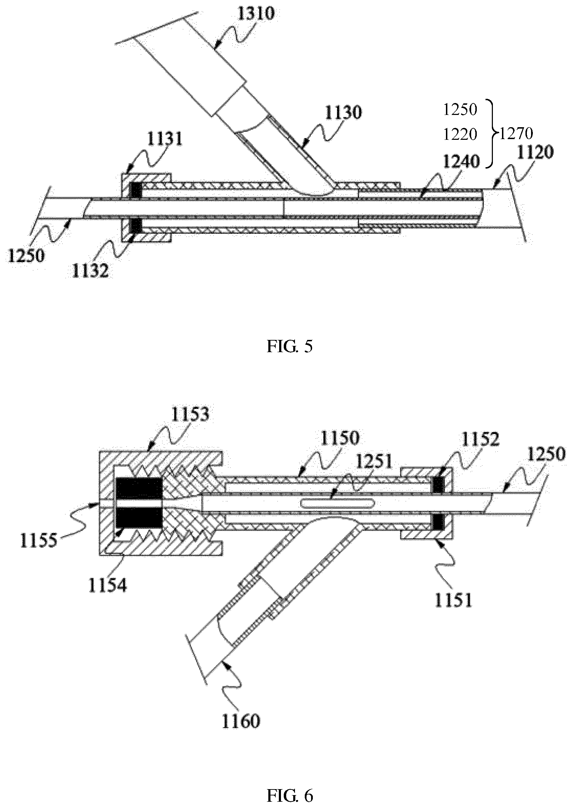

FIG. 6 is a schematic cross-sectional diagram of the rear-end connector of the transmission tube. As shown in FIG. 6, to ensure that the transmission tube 1270 extends and is fixed in the rear-end connector 1150 of the transmission tube in a stable and airtight manner, a distal end of the rear-end connector 1150 of the transmission tube is fixed on the transmission tube 1270 through cooperation of a fixing cap 1151 and a seal ring 1152 (a rear-end transmission tube 1250 of the transmission tube 1270 is merely shown); and a proximal end of the rear-end connector 1150 of the transmission tube is locked and fixed externally from the tip of the transmission tube 1270 through cooperation of a locking cap 1153 and a seal collar 1154. The proximal end of the rear-end connector 1150 of the transmission tube and a proximal end of the locking cap 1153 are each provided with a guidewire hole 1155 which serves as a passageway for a guidewire and is communicated with the transmission tube 1270. Preferably, the locking cap 1153 and the rear-end connector 1150 of the transmission tube are fixed through fitting threads, and the locking cap 1153 is tightened or loosened to close or open the guidewire hole 1155. When the locking cap 1153 is screwed tightly, the guidewire hole 1155 is closed, and the tip of the transmission tube 1270 is sealed. When the locking cap 1153 is loosened, the guidewire hole 1155 is opened to let a guidewire pass.

As shown in FIG. 6, the transmission tube 1270 that extends into the rear-end connector 1150 of the transmission tube has at least one perfusion hole 1251, through which the thrombolytic drug in the infusion tube 1160 can enter the transmission tube 1270.

The working processes of the thrombus aspiration system, the thrombus fragmentation system, and the drug perfusion system in the mechanical thrombectomy apparatus 1000 of the present invention are described as follows.

The working process of the thrombus aspiration system is that: the aspiration device (namely, the peristaltic pump 1340) produces a negative pressure → the thrombus removal head 1110 aspirates a thrombus under the negative pressure → the thrombus enters the lumen of the sheath 1120 → the thrombus is delivered to the proximal end through the thrombus aspiration tube (namely, the flexible tube 1310) → the collection vessel 1330 collects the thrombus.

The working process of the thrombus fragmentation system is that: the rotation actuator 1140 starts working → the transmission tube 1270 rotates → the thrombus fragmentation cutter 1210 is driven to rotate → a thrombus aspirated into the thrombus removal head 1110 is fragmented.

The working process of the drug perfusion system is that: a thrombolytic drug is loaded into the injector 1180 → the infusion tube 1160 delivers the thrombolytic drug → the transmission tube 1270 continues to deliver the thrombolytic drug → the thrombolytic drug is discharged from the thrombus removal head 1110 → the thrombolytic drug reaches the site of a thrombus for softening or dissolving the thrombus.

The mechanical thrombectomy apparatus of the present invention integrates drug perfusion, thrombus aspiration, and mechanical thrombus fragmentation. It can be known from the working process of each system that, a thrombolytic drug is perfused through a lumen (that is, the transmission tube) which is independent from thrombus aspiration (carried out in the sheath). Hence, at the same time as or before thrombus fragmentation and aspiration, the thrombolytic drug can be perfused to the site of a thrombus for dissolving or softening the thrombus. Therefore, thrombi that block blood vessels can be expelled through aspiration as much as possible in the cases of acute and subacute thrombosis, and the reduction of the thrombectomy effect caused by the circumstance that the thrombi again enter the blood vessels along with perfusion can be avoided. In addition, the thrombolytic drug can be perfused into the blood vessels after thrombus fragmentation and aspiration, such that residual thrombi in a small quantity are dissolved and the recurrent rate of thrombosis is lowered.

In the present embodiment, the specific structure of the rotation actuator 1140 includes, but not limited to, the following two configurations.

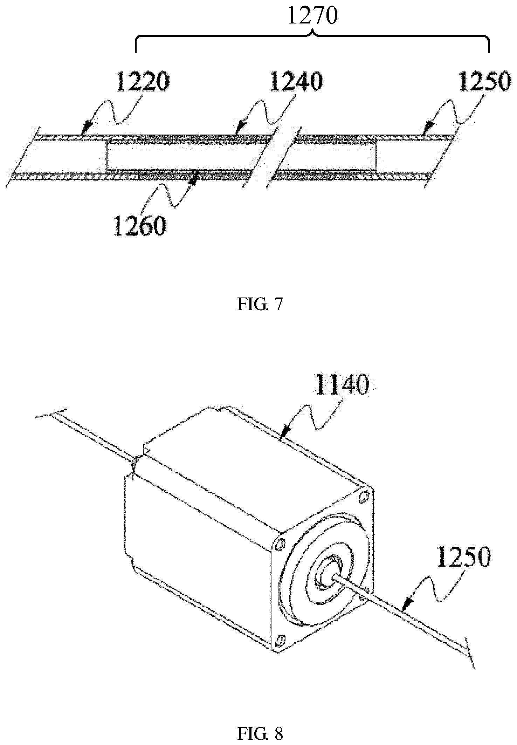

Configuration 1: As shown in FIG. 8, the rotation actuator 1140 is a hollow shaft motor mounted on an external side of the transmission tube 1270 (the rear-end transmission tube 1250 is merely shown). In other words, the transmission tube 1270 serves as a rotary shaft of the hollow shaft motor. When the hollow shaft motor runs, the transmission tube 1270 is enabled to rotate and the thrombus fragmentation cutter 1210 fixed on the distal end of the transmission tube 1270 is driven to rotate accordingly, thereby realizing fragmentation of thrombi around the thrombus fragmentation cutter 1210.

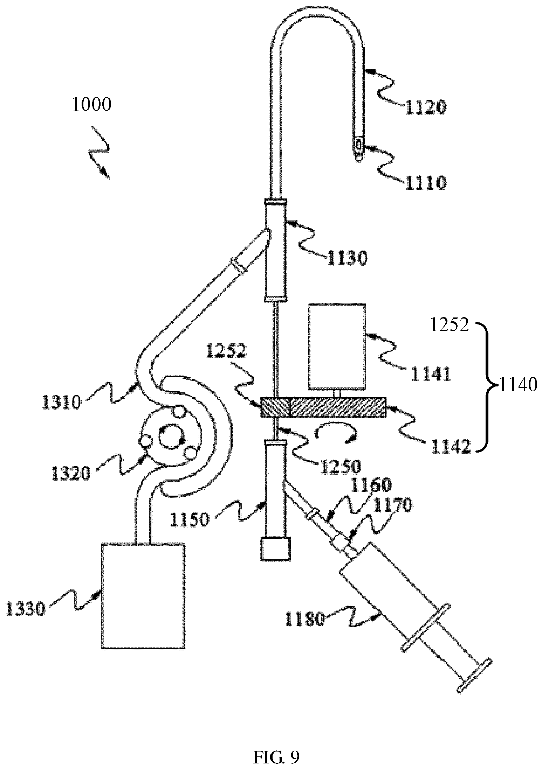

Configuration 2: As shown in FIG. 9, the rotation actuator 1140 includes: a rotary motor 1141, a first gear 1252, and a second gear 1142. The first gear 1252 is mounted on the external side of the transmission tube 1270 (the transmission tube 1270 serves as a shaft of the first gear 1252) and is meshed with the second gear 1142. The second gear 1142 is mounted on an output shaft of the rotary motor 1141. When the rotary motor 1141 starts working, its output shaft rotates and drives the second gear 1142 to rotate. Since the first gear 1252 is meshed with the second gear 1142, the first gear 1252 is driven to rotate along with the second gear 1142, the transmission tube 1270 is enabled to rotate accordingly, and thus the thrombus fragmentation cutter 1210 fixed on the distal end of the transmission tube 1270 is forced to rotate, thereby realizing fragmentation of thrombi that enter the thrombus removal head 1110 and are around the thrombus fragmentation cutter 1210.

Referring to FIG. 1 and FIG. 5, FIG. 5 is a schematic cross-sectional diagram of the sheath connector. The sheath connector 1130 is in a triple lumen structure, including a distal lumen, a proximal lumen, and a side lumen. The transmission tube 1270 passes through the distal lumen and the proximal lumen of the sheath connector 1130 (that is, the transmission tube 1270 enters the lumen of the sheath 1120 through the sheath connector 1130), and the side lumen of the sheath connector 1130 is connected to the distal end of the thrombus aspiration tube (namely, the flexible tube 1310 in FIG. 1). Each of the systems (which comprise the thrombus removal system, the thrombus fragmentation system, and the drug perfusion system) is communicated with the sheath 1120 through the sheath connector 1130 which functions as an adapter. In the present embodiment, the thrombus removal system and the thrombus fragmentation system are connected through the sheath connector 1130, and the thrombus fragmentation system and the drug perfusion system are connected through the rear-end connector 1150 of the transmission tube. However, it is apparent to persons skilled in the art that the foregoing connection manner is merely a preferred connection manner, and the thrombus removal system, the thrombus fragmentation system, and the drug perfusion system can all be connected through the sheath connector 1130.

As shown in FIG. 3, the transmission tube 1270 is in a hollow tubular structure, through which a guidewire is delivered and a thrombolytic drug is perfused. The distal end of the transmission tube 1270 includes a first side hole 1221 which matches with an inclined hole 1112 on the thrombus removal head 1110. The drug passes through the first side hole 1221 and the inclined hole 1112 to reach the site of a thrombus.

As shown in FIG. 5, the sheath connector 1130 further includes a seal structure for sealing the thrombus aspiration lumen (that is, a lumen formed by connecting a cavity defined by the transmission tube and the sheath to the thrombus aspiration tube). The seal structure includes a fixing cap 1131 and a seal ring 1132. The transmission tube 1270 that extends and passes through the sheath connector 1130 is fixed to the sheath connector 1130 through cooperation of the fixing cap 1131 and the seal ring 1132.

In the present embodiment, the transmission tube 1270 includes: a front-end transmission tube 1220, an intermediate transmission tube 1240, and a rear-end transmission tube 1250 that are sequentially arranged and communicated with each other. The thrombus fragmentation cutter 1210 is fixed on the front-end transmission tube 1220 and is accommodated in the thrombus removal head. The intermediate transmission tube 1240 is accommodated in the sheath 1120 and the sheath connector 1130. The rear-end transmission tube 1250 is connected to the rotation actuator 1140. Specifically, the at least one first side hole 1221 is provided on the circumference of the front-end transmission tube 1220; and the at least one perfusion hole 1251 is provided on the part of the rear-end transmission tube 1250 that extends and is fixed in the rear-end connector 1150 of the transmission tube. The front-end transmission tube 1220 is preferably a fixed tube for transmitting rotation. The intermediate transmission tube 1240 is a spiral tube, which is flexible to some extent to relieve a rotating force transmitted by the rear-end transmission tube 1250, thereby avoiding breakage of the tube. To enhance the airtightness between the sections of the transmission tube 1270, an inner tube 1260 is provided in the spiral tube. The inner tube 1260 covers the connection parts between the spiral tube, the front-end transmission tube 1220, and the rear-end transmission tube 1250 (as shown in FIG. 7).

Referring to FIG. 2 and FIG. 3, the thrombus removal head 1110 is a hollow member with a proximal end and a distal end. The proximal end of the thrombus removal head includes at least one thrombus removal side hole 1113, and is preferably in an annular structure. The distal end of the thrombus removal head is in a tapered structure and has at least one inclined hole 1112 and a top hole 1111. A thrombus enters the thrombus removal head 1110 via the thrombus removal side hole 1113. The thrombus fragmentation cutter 1210 is located in the lumen of the thrombus removal head 1110, and the location of the blade 1211 is corresponding to the location of the thrombus removal side hole 1113. The thrombolytic drug is perfused via the inclined hole 1112 and the top hole 1111 on the thrombus removal head. Compared with the delivery of the thrombolytic drug to a thrombus merely via merely the top hole 1111, the presence of multiple inclined holes 1112 can effectively increase the contact area between the thrombolytic drug and the thrombus. The inclined hole 1112 is formed along a direction oblique to the surface of the tapered structure, which increases the contact area between the perfused thrombolytic drug and the thrombus and improves the thrombolysis efficiency as compared with a manner of forming a hole along a direction perpendicular to the surface of the tapered structure.

Further referring to FIG. 3, the sheath 1120 is connected to the thrombus removal head 1110 through a positioning ring 1230 which is in a hollow tubular structure. The positioning ring 1230 sleeves over and fixed on the external side of the transmission tube 1270 and is close to the thrombus fragmentation cutter 1210, to limit the position of the thrombus fragmentation cutter 1210. The sheath 1120 is made of a single-layer tubular material or a multilayer composite tubular material. The layered structure of the sheath 1120 made of a multilayer composite tubular material includes a metallic braided layer.

During fabrication of each component of the mechanical thrombectomy apparatus in the present embodiment, each component can be processed by means of machining, injection (extrusion) molding, and/or laser engraving. Different components can be fixedly connected by means of laser welding or adhesion. For example, the front-end transmission tube 1220, the intermediate transmission tube 1240, and the rear-end transmission tube 1250 are sequentially welded by laser to form the transmission tube 1270. The thrombus fragmentation cutter 1210 is fixed on the front-end transmission tube 1220 by means of laser welding. The flexible tube is mounted on the sheath connector 1130 by adhesion. The foregoing content is not related to improvements of the technical solution of the present invention and no further description is made herein.

To better understand the mechanical thrombectomy apparatus of the present invention, the application of the mechanical thrombectomy apparatus 1000 will be described in detail below with reference to FIG. 1. The specific process is as follows.

Before an operation starts, the thrombus removal head 1110 of the mechanical thrombectomy apparatus 1000 is placed in saline, the peristaltic pump 1340 is started and works till saline can be found in the collection vessel 1330, the injector 1180 is connected to the check valve 1170 such that the injector 1180 is connected to the infusion tube 1160, aspiration is started and lasts till saline can be found in the injector 1180, and thus the mechanical thrombectomy apparatus 1000 is evacuated.

During the operation, the thrombus removal head 1110 and at least a part of the sheath 1120 in the mechanical thrombectomy apparatus 1000 are delivered to the site of a lesion (thrombus) along a guidewire (purchased separately) through a puncture in a blood vessel. The locking cap 1153 is screwed tightly. The rotation actuator 1140, the aspiration device (namely, the peristaltic pump 1340), the injector 1180 and the like are placed outside the body. The injector 1180 draws a thrombolytic drug (for example, urokinase) and then is communicated with the check valve 1170. By pressing the injector 1180, the thrombolytic drug passes through the infusion tube 1160 to reach the rear-end connector 1150 of the transmission tube, and enters the lumen of the transmission tube 1270 via the perfusion hole 1251. Then, the thrombolytic drug sequentially passes through the lumens of the rear-end transmission tube 1250, the intermediate transmission tube 1240, and the front-end transmission tube 1220, and reaches the site of the lesion (thrombus) via the first side hole 1221 as well as the top hole 1111 and the inclined hole 1112 on the thrombus removal head. The thrombolytic drug contacts the thrombus and softens or dissolves the thrombus. In about 10 minutes, the rotation actuator 1140 is started, and the transmission tube 1270 drives the thrombus fragmentation cutter 1210 to rotate. After that, the peristaltic pump is started, the flexible tube 1310 is squeezed to exert a suction force on the lumen of the sheath 1120, and thus a negative pressure is produced to aspirate the thrombus into the thrombus removal head 1110 via the thrombus removal side hole 1113. The rotating blade 1211 and the fixed thrombus removal side hole 1113 cooperate to fragment the thrombus aspirated into the thrombus removal head 1110. The thrombus is then expelled through aspiration under the negative pressure provided by the peristaltic pump 1340 and is delivered into the collection vessel 1330. During the operation, the thrombus removal head 1110 of the mechanical thrombectomy apparatus 1000 can be moved forward or backward along the guidewire according to the specific position of the thrombus in the blood vessel, thereby improving the thrombus removal efficiency; meanwhile, the thrombolytic drug can be perfused to the site of the thrombus via the injector 1180 whenever needed.

After thrombus aspiration, the rotation actuator 1140 and the peristaltic pump are turned off in sequence, and finally all the components are pulled out of the body.

In view of the above, the mechanical thrombectomy apparatus has the following beneficial effects:
(1) The thrombus aspiration system provides a negative pressure in a manner different from an existing manner of generating a negative pressure via siphoning by high-speed rotation of a spiral structure. Thus, the negative pressure required for thrombus aspiration can be generated without high-speed rotation. In this way, the problem of breakage of a guidewire caused by over-high temperature resulting from extremely high-speed rotation of the spiral structure in the existing process of generating a negative pressure can be effectively solved.
(2) The apparatus integrates perfusion of a thrombolytic drug, thrombus aspiration by a negative pressure, and mechanical thrombus fragmentation, which can be carried out at the same time to significantly accelerate the thrombectomy procedure, reduce the operation time, and relieve pain from the patient.
(3) It is convenient for doctors to choose one or a combination of the above modes for thrombectomy according to the condition of the patient during the operation. Therefore, the application range of the mechanical thrombectomy apparatus is expanded.
(4) The perfusion of the thrombolytic drug is carried out in the lumen of the transmission tube independent from thrombus aspiration. Hence, the thrombolytic drug can be perfused to the site of a lesion at any time during the operation. Therefore, the problem caused by the circumstance that the thrombus already aspirated into the thrombus removal head again enters the body of the patient along with drug perfusion can be avoided.
(5) Since thrombus fragmentation is carried out in the thrombus removal head, the blood cells will not be destroyed, and the probability of incurring hematuria and renal failure complications are largely reduced.
(6) The thrombus fragmentation cutter is located in the lumen of the thrombus removal head, which can effectively reduce the incidence of vascular perforation.

The above descriptions are merely preferred embodiments of the present invention, and form no limitations to the scope of the present invention. The protection scope of the present invention is defined by the appended claims.

## Claims

1. A mechanical thrombectomy apparatus (1000), comprising:
a thrombus removal head (1110), connected to a sheath connector (1130) through a sheath (1120);
a thrombus fragmentation system, comprising a rotation actuator (1140), a transmission tube (1270), and a thrombus fragmentation cutter (1210), wherein a proximal end of the transmission tube (1270) is connected to the rotation actuator (1140), a distal end of the transmission tube (1270) is connected to the thrombus fragmentation cutter (1210), and the transmission tube (1270) extends into the thrombus removal head (1110) along lumens of the sheath connector (1130) and the sheath (1120); when driven by the rotation actuator (1140), the transmission tube (1270) is able to rotate and drive the thrombus fragmentation cutter (1210) to rotate, so as to fragment a thrombus aspirated into the thrombus removal head (1110);
a thrombus aspiration system, comprising an aspiration device and a thrombus aspiration tube, wherein a proximal end of the thrombus aspiration tube is connected to the aspiration device, and a distal end of the thrombus aspiration tube is connected to the sheath connector (1130); a cavity formed between the transmission tube (1270) and the sheath (1120) is communicated with the thrombus aspiration tube to form a thrombus aspiration lumen; and
a drug perfusion system, comprising an injector (1180) and an infusion tube (1160), a proximal end of the infusion tube (1160) is connected to the injector (1180), a distal end of the infusion tube (1160) is communicated with the transmission tube (1270) to allow a drug to pass through the transmission tube (1270) and the thrombus removal head (1110) to reach a site of a thrombus;
wherein the aspiration device is a peristaltic pump (1340), and the thrombus aspiration tube is a flexible tube (1310); and
wherein the thrombus removal head (1110) has a distal end that has a tapered structure and has at least one inclined hole (1112) and a top hole (1111), and the inclined hole (1112) and the top hole (1111) are configured so that the drug is allowed be perfused therethrough; and
wherein the top hole (1111) is formed at a tip of the tapered structure.

2. The mechanical thrombectomy apparatus (1000) according to claim 1, wherein the rotation actuator (1140) is a hollow shaft motor mounted on an external side of the transmission tube (1270); or
the rotation actuator (1140) comprises: a rotary motor (1141), a first gear (1252), and a second gear (1142), the first gear (1252) is mounted on an external side of the transmission tube (1270) and is meshed with the second gear (1142), and the second gear (1142) is mounted on an output shaft of the rotary motor (1141).

3. The mechanical thrombectomy apparatus (1000) according to claim 1, wherein the sheath connector (1130) is in a triple lumen structure, the transmission tube (1270) passes through a distal lumen and a proximal lumen of the sheath connector (1130), a side lumen of the sheath connector (1130) is connected to the distal end of the thrombus aspiration tube, and the sheath connector (1130) further comprises a seal structure configured to seal the thrombus aspiration lumen.

4. The mechanical thrombectomy apparatus (1000) according to claim 2, wherein the distal end of the infusion tube (1160) is communicated with the transmission tube (1270) through the sheath connector (1130), or the distal end of the infusion tube (1160) is communicated with the transmission tube (1270) through a rear-end connector (1150) of the transmission tube (1270).

5. The mechanical thrombectomy apparatus (1000) according to claim 1, wherein the transmission tube (1270) comprises: a front-end transmission tube (1220), an intermediate transmission tube (1240), and a rear-end transmission tube (1250) that are sequentially arranged and communicated with each other, the thrombus fragmentation cutter (1210) is fixed on the front-end transmission tube (1220) and is accommodated in the thrombus removal head (1110), the intermediate transmission tube (1240) is accommodated in the sheath (1120) and the sheath connector (1130), and the rear-end transmission tube (1250) is connected to the rotation actuator (1140).

6. The mechanical thrombectomy apparatus (1000) according to claim 5, wherein the intermediate transmission tube (1240) is a spiral tube, an inner tube (1260) is provided in the spiral tube, and the inner tube (1260) covers connection parts between the spiral tube, the front-end transmission tube (1220), and the rear-end transmission tube (1250).

7. The mechanical thrombectomy apparatus (1000) according to claim 4, wherein a distal end of the rear-end connector (1150) of the transmission tube (1270) is fixed on the transmission tube (1270) through cooperation of a fixing cap (1151) and a seal ring (1152), a proximal end of the rear-end connector (1150) of the transmission tube (1270) is fixed on the distal end of the transmission tube (1270) through cooperation of a locking cap (1153) and a seal collar (1154), and the proximal end of the rear-end connector (1150) of the transmission tube (1270) and a proximal end of the locking cap (1153) are each provided with a guidewire hole (1155) which serves as a passageway for a guidewire and is communicated with the transmission tube (1270).

8. The mechanical thrombectomy apparatus (1000) according to claim 7, wherein the locking cap (1153) and the rear-end connector (1150) of the transmission tube (1270) are provided with fitting threads, through which the locking cap (1153) is tightened or loosened to close or open the guidewire hole (1155).

9. The mechanical thrombectomy apparatus (1000) according to claim 1, wherein the thrombus removal head (1110) is a hollow member, the distal end of the thrombus removal head (1110) is in a tapered structure, and a proximal end of the thrombus removal head (1110) comprises at least one thrombus removal side hole (1113).

10. The mechanical thrombectomy apparatus (1000) according to claim 9, wherein the thrombus fragmentation cutter (1210) is in a hollow-out tubular structure and comprises at least one blade (1211), the thrombus fragmentation cutter (1210) is placed in a lumen of the thrombus removal head (1110), and the blade (1211) is in positional correspondence to the thrombus removal side hole (1113).

11. The mechanical thrombectomy apparatus (1000) according to claim 1, wherein the distal end of the transmission tube (1270) comprises a first side hole (1221) which matches with the inclined hole (1112) on the thrombus removal head (1110) to allow the drug to pass through the first side hole (1221) and the inclined hole (1112) to reach the site of the thrombus.

## Patentansprüche

1. Mechanisches Thrombektomie-Gerät (1000), umfassend:
einen Thrombus-Entfernungskopf (1110), der über einen Schaft (1120) mit einem Schaftverbinder (1130) verbunden ist;
ein Thrombus-Fragmentierungssystem, umfassend einen Drehaktuator (1140), ein Übertragungsrohr (1270) und eine Thrombus-Fragmentierungsschneideinrichtung (1210), wobei ein proximales Ende des Übertragungsrohrs (1270) mit dem Drehaktuator (1140) verbunden ist, ein distales Ende des Übertragungsrohrs (1270) mit der Thrombus-Fragmentierungsschneideinrichtung (1210) verbunden ist und das Übertragungsrohr (1270) sich entlang von Lumen des Schaftverbinders (1130) und des Schafts (1120) in den Thrombus-Entfernungskopf (1110) erstreckt; das Übertragungsrohr (1270), wenn es durch den Drehaktuator (1140) angetrieben wird, in der Lage ist, zu drehen und die Thrombus-Fragmentierungsschneideinrichtung (1210) anzutreiben, um einen in den Thrombus-Entfernungskopf (1110) angesaugten Thrombus zu fragmentieren;
ein Thrombus-Absaugsystem, umfassend eine Absaugvorrichtung und ein Thrombus-Absaugrohr, wobei ein proximales Ende des Thrombus-Absaugrohrs mit der Absaugvorrichtung verbunden ist und ein distales Ende des Thrombus-Absaugrohrs mit dem Schaftverbinder (1130) verbunden ist; ein zwischen dem Übertragungsrohr (1270) und dem Schaft (1120) gebildeter Hohlraum mit dem Thrombus-Absaugrohr in Verbindung ist, um ein Thrombus-Absauglumen zu bilden; und
ein Medikamenten-Perfusionssystem, umfassend einen Injektor (1180) und ein Infusionsrohr (1160), wobei ein proximales Ende des Infusionsrohrs (1160) mit dem Injektor (1180) verbunden ist, ein distales Ende des Infusionsrohrs (1160) mit dem Übertragungsrohr (1270) in Verbindung ist, um zu ermöglichen, dass ein Medikament durch das Übertragungsrohr (1270) und den Thrombus-Entfernungskopf (1110) verläuft, um eine Stelle eines Thrombus zu erreichen;
wobei die Absaugvorrichtung eine peristaltische Pumpe (1340) ist und das Thrombus-Absaugrohr ein Schlauch (1310) ist; und
wobei der Thrombus-Entfernungskopf (1110) ein distales Ende aufweist, das eine verjüngte Struktur aufweist und mindestens ein geneigtes Loch (1112) und ein oberes Loch (1111) aufweist, und das geneigte Loch (1112) und das obere Loch (1111) konfiguriert sind, sodass das Medikament durch sie hindurch perfundiert werden kann; und
wobei das obere Loch (1111) an einer Spitze der sich verjüngenden Struktur gebildet ist.

2. Mechanisches Thrombektomie-Gerät (1000) nach Anspruch 1, wobei der Drehaktuator (1140) ein Hohlwellenmotor ist, der an einer Außenseite des Übertragungsrohrs (1270) montiert ist; oder
der Drehaktuator (1140) Folgendes umfasst: einen Drehmotor (1141), ein erstes Zahnrad (1252) und ein zweites Zahnrad (1142), wobei das erste Zahnrad (1252) an einer Außenseite des Übertragungsrohrs (1270) montiert ist und mit dem zweiten Zahnrad (1142) in Eingriff ist, und das zweite Zahnrad (1142) an einer Ausgangswelle des Drehmotors (1141) montiert ist.

3. Mechanisches Thrombektomie-Gerät (1000) nach Anspruch 1, wobei der Schaftverbinder (1130) eine Dreilumenstruktur aufweist, das Übertragungsrohr (1270) durch ein distales Lumen und ein proximales Lumen des Schaftverbinders (1130) verläuft, ein Seitenlumen des Schaftverbinders (1130) mit dem distalen Ende des Thrombus-Absaugrohrs verbunden ist und der Schaftverbinder (1130) ferner eine Dichtungsstruktur umfasst, die konfiguriert ist, um das Thrombus-Absauglumen abzudichten.

4. Mechanisches Thrombektomie-Gerät (1000) nach Anspruch 2, wobei das distale Ende des Infusionsrohrs (1160) durch den Schaftverbinder (1130) mit dem Übertragungsrohr (1270) verbunden ist, oder das distale Ende des Infusionsrohrs (1160) durch einen hinteren Endverbinder (1150) des Übertragungsrohrs (1270) mit dem Übertragungsrohr (1270) verbunden ist.

5. Mechanisches Thrombektomie-Gerät (1000) nach Anspruch 1, wobei das Übertragungsrohr (1270) Folgendes umfasst: ein vorderes Übertragungsrohr (1220), ein mittleres Übertragungsrohr (1240) und ein hinteres Übertragungsrohr (1250), die nacheinander angeordnet sind und miteinander in Verbindung sind, wobei die Thrombus-Fragmentierungsschneideinrichtung (1210) an dem vorderen Übertragungsrohr (1220) befestigt ist und in dem Thrombus-Entfernungskopf (1110) untergebracht ist, das Zwischenübertragungsrohr (1240) in dem Schaft (1120) und dem Schaftverbinder (1130) untergebracht ist, und das hintere Übertragungsrohr (1250) mit dem Drehantrieb (1140) verbunden ist.

6. Mechanisches Thrombektomie-Gerät (1000) nach Anspruch 5, wobei das Zwischenübertragungsrohr (1240) ein Spiralrohr ist, ein Innenrohr (1260) in dem Spiralrohr bereitgestellt ist und das Innenrohr (1260) Verbindungsteile zwischen dem Spiralrohr, dem vorderen Übertragungsrohr (1220) und dem hinteren Übertragungsrohr (1250) abdeckt.

7. Mechanisches Thrombektomie-Gerät (1000) nach Anspruch 4, wobei ein distales Ende des hinteren Verbinders (1150) des Übertragungsrohrs (1270) durch Zusammenwirken einer Befestigungskappe (1151) und eines Dichtungsrings (1152) an dem Übertragungsrohr (1270) befestigt ist, ein proximales Ende des hinteren Verbinders (1150) des Übertragungsrohrs (1270) durch Zusammenwirken einer Verriegelungskappe (1153) und eines Dichtungsrings (1154) an dem distalen Ende des Übertragungsrohrs (1270) befestigt ist, und das proximale Ende des hinteren Verbinders (1150) des Übertragungsrohrs (1270) und ein proximales Ende der Verriegelungskappe (1153) jeweils mit einem Führungsdrahtloch (1155) versehen sind, das als Durchgang für einen Führungsdraht dient und mit dem Übertragungsrohr (1270) in Verbindung ist.

8. Mechanisches Thrombektomie-Gerät (1000) nach Anspruch 7, wobei die Verriegelungskappe (1153) und der hintere Verbinder (1150) des Übertragungsrohrs (1270) mit Passgewinden versehen sind, durch die die Verriegelungskappe (1153) angezogen oder gelöst wird, um das Führungsdrahtloch (1155) zu schließen oder zu öffnen.

9. Mechanisches Thrombektomie-Gerät (1000) nach Anspruch 1, wobei der Thrombus-Entfernungskopf (1110) ein Hohlelement ist, das distale Ende des Thrombus-Entfernungskopfs (1110) eine sich verjüngende Struktur aufweist und ein proximales Ende des Thrombus-Entfernungskopfs (1110) mindestens ein Thrombusentfernung-Seitenloch (1113) umfasst.

10. Mechanisches Thrombektomie-Gerät (1000) nach Anspruch 9, wobei die Thrombus-Fragmentierungsschneideinrichtung (1210) in einer hohlen, röhrenförmigen Struktur ist und mindestens eine Klinge (1211) umfasst, wobei die Thrombus-Fragmentierungsschneideinrichtung (1210) in einem Lumen des Thrombus-Entfernungskopfs (1110) platziert ist und die Klinge (1211) in Positionskorrespondenz zu dem Thrombusentfernung-Seitenloch (1113) ist.

11. Mechanisches Thrombektomie-Gerät (1000) nach Anspruch 1, wobei das distale Ende des Übertragungsrohrs (1270) ein erstes Seitenloch (1221) aufweist, das mit dem geneigten Loch (1112) an dem Thrombus-Entfernungskopf (1110) zusammenpasst, um zu ermöglichen, dass das Medikament durch das erste Seitenloch (1221) und das geneigte Loch (1112) verläuft, um die Stelle des Thrombus zu erreichen.

## Revendications

1. Appareil mécanique de thrombectomie (1000), comprenant :
une tête d'élimination de thrombus (1110), connectée à un connecteur de gaine (1130) par l'intermédiaire d'une gaine (1120) ;
système de fragmentation de thrombus, comprenant un actionneur de rotation (1140), un tube de transmission (1270) et un dispositif de coupe de fragmentation de thrombus (1210), dans lequel une extrémité proximale du tube de transmission (1270) est reliée à l'actionneur de rotation (1140), une extrémité distale du tube de transmission (1270) est reliée au dispositif de coupe de fragmentation de thrombus (1210), et le tube de transmission (1270) s'étend dans la tête d'élimination de thrombus (1110) le long de la lumière du connecteur de gaine (1130) et de la gaine (1120) ; lorsqu'il est entraîné par l'actionneur de rotation (1140), le tube de transmission (1270) est capable de tourner et d'entraîner le dispositif de coupe de fragmentation de thrombus (1210) en rotation, de manière à fragmenter un thrombus aspiré dans la tête d'élimination de thrombus (1110) ;
un système d'aspiration de thrombus, comprenant un dispositif d'aspiration et un tube d'aspiration de thrombus, dans lequel une extrémité proximale du tube d'aspiration de thrombus est reliée au dispositif d'aspiration, et une extrémité distale du tube d'aspiration de thrombus est reliée au connecteur de gaine (1130) ; une cavité formée entre le tube de transmission (1270) et la gaine (1120) est mise en communication avec le tube d'aspiration de thrombus pour former une lumière d'aspiration de thrombus ; et
système de perfusion de médicament, comprenant un injecteur (1180) et un tube de perfusion (1160), une extrémité proximale du tube de perfusion (1160) est reliée à l'injecteur (1180), une extrémité distale du tube de perfusion (1160) est mise en communication avec le tube de transmission (1270) pour permettre à un médicament de passer à travers le tube de transmission (1270) et la tête d'élimination de thrombus (1110) pour atteindre le site d'un thrombus ;
dans lequel le dispositif d'aspiration est une pompe péristaltique (1340), et le tube d'aspiration de thrombus est un tube flexible (1310) ; et
dans lequel la tête d'élimination de thrombus (1110) a une extrémité distale qui a une structure conique et a au moins un trou incliné (1112) et un trou supérieur (1111), et le trou incliné (1112) et le trou supérieur (1111) sont configurés de sorte que le médicament peut être perfusé à travers ceux-ci ; et
dans lequel le trou supérieur (1111) est formé à une extrémité de la structure conique.

2. Appareil de thrombectomie mécanique (1000) selon la revendication 1, dans lequel l'actionneur de rotation (1140) est un moteur à arbre creux monté sur un côté externe du tube de transmission (1270) ; ou
l'actionneur de rotation (1140) comprend : un moteur rotatif (1141), un premier engrenage (1252), et un second engrenage (1142), le premier engrenage (1252) est monté sur un côté externe du tube de transmission (1270) et est engrené avec le second engrenage (1142), et le second engrenage (1142) est monté sur un arbre de sortie du moteur rotatif (1141).

3. Appareil de thrombectomie mécanique (1000) selon la revendication 1, dans lequel le connecteur de gaine (1130) se trouve dans une structure à triple lumière, le tube de transmission (1270) passe à travers une lumière distale et une lumière proximale du connecteur de gaine (1130), une lumière latérale du connecteur de gaine (1130) est reliée à l'extrémité distale du tube d'aspiration de thrombus, et le connecteur de gaine (1130) comprend en outre une structure d'étanchéité configurée pour rendre étanche la lumière d'aspiration de thrombus.

4. Appareil de thrombectomie mécanique (1000) selon la revendication 2, dans lequel l'extrémité distale du tube de perfusion (1160) est mise en communication avec le tube de transmission (1270) par l'intermédiaire du connecteur de gaine (1130), ou l'extrémité distale du tube de perfusion (1160) est mise en communication avec le tube de transmission (1270) par l'intermédiaire d'un connecteur d'extrémité arrière (1150) du tube de transmission (1270).

5. Appareil de thrombectomie mécanique (1000) selon la revendication 1, dans lequel le tube de transmission (1270) comprend : un tube de transmission d'extrémité avant (1220), un tube de transmission intermédiaire (1240), et un tube de transmission d'extrémité arrière (1250) qui sont agencés successivement et communiquent les uns avec les autres, le dispositif de coupe de fragmentation de thrombus (1210) est fixé sur le tube de transmission d'extrémité avant (1220) et est logé dans la tête d'élimination de thrombus (1110), le tube de transmission intermédiaire (1240) est logé dans la gaine (1120) et le connecteur de gaine (1130), et le tube de transmission arrière (1250) est relié à l'actionneur de rotation (1140).

6. Appareil de thrombectomie mécanique (1000) selon la revendication 5, dans lequel le tube de transmission intermédiaire (1240) est un tube en spirale, un tube interne (1260) est prévu dans le tube en spirale, et le tube interne (1260) couvre les parties de connexion entre le tube en spirale, le tube de transmission d'extrémité avant (1220), et le tube de transmission d'extrémité arrière (1250).

7. Appareil de thrombectomie mécanique (1000) selon la revendication 4, dans lequel une extrémité distale du connecteur d'extrémité arrière (1150) du tube de transmission (1270) est fixée sur le tube de transmission (1270) par coopération d'un capuchon de fixation (1151) et d'une bague d'étanchéité (1152), une extrémité proximale du connecteur d'extrémité arrière (1150) du tube de transmission (1270) est fixée sur l'extrémité distale du tube de transmission (1270) par coopération d'un capuchon de verrouillage (1153) et d'un collier d'étanchéité (1154), et l'extrémité proximale du connecteur d'extrémité arrière (1150) du tube de transmission (1270) et une extrémité proximale du capuchon de verrouillage (1153) sont chacune munies d'un trou de fil guide (1155) qui sert de passage pour un fil guide et communique avec le tube de transmission (1270).

8. Appareil de thrombectomie mécanique (1000) selon la revendication 7, dans lequel le capuchon de verrouillage (1153) et le connecteur d'extrémité arrière (1150) du tube de transmission (1270) sont munis d'un filetage, à travers lequel le capuchon de verrouillage (1153) est serré ou desserré pour fermer ou ouvrir le trou de fil guide (1155).

9. Appareil de thrombectomie mécanique (1000) selon la revendication 1, dans lequel la tête d'élimination de thrombus (1110) est un élément creux, l'extrémité distale de la tête d'élimination de thrombus (1110) présente une structure conique, et une extrémité proximale de la tête d'élimination de thrombus (1110) comprend au moins un trou latéral d'élimination de thrombus (1113).

10. Appareil de thrombectomie mécanique (1000) selon la revendication 9, dans lequel le dispositif de coupe de fragmentation de thrombus (1210) présente une structure tubulaire évidée et comprend au moins une lame (1211), le dispositif de coupe de fragmentation de thrombus (1210) est placé dans une lumière de la tête d'élimination de thrombus (1110), et la lame (1211) est en correspondance positionnelle avec le trou latéral d'élimination de thrombus (1113).

11. Appareil de thrombectomie mécanique (1000) selon la revendication 1, dans lequel l'extrémité distale du tube de transmission (1270) comprend un premier trou latéral (1221) qui correspond au trou incliné (1112) sur la tête d'élimination de thrombus (1110) pour permettre au médicament de passer à travers le premier trou latéral (1221) et le trou incliné (1112) pour atteindre le site du thrombus.
